# EUROPEAN PATENT APPLICATION

(11) **EP 1 428 497 A1**
(43) Date of publication of application: **16.06.2004**
(21) Application number: 02027801.6
(22) Date of filing: 12.12.2002
(51) Int. Cl.: A61K 7/06

(54) **Pre-treatment composition applied before colouring hair with acidic direct dyes**

(71) Applicant: KPSS-Kao Professional Salon Services GmbH, 64297 Darmstadt (DE)
(72) Inventor: Dürr, Manfred, 64389 Münster (DE); Shinozaki, Takao, 60439 Frankfurt (DE); Nöcker, Bernd, Dr., 64372 Ober-Ramstadt (DE)

(57) **Abstract**

SUMMARY

This invention relates to a pre-treatment composition and to a process for application of the said composition, which has an acidic pH and is applied to hair, especially damaged hair, before colouring hair with colouring agents containing acidic, anionic, direct dyes in order to improve the colour intensity and at the same time to achieve durable hair colour. pH of the pre-treatment compositions is from 1.5 to 5. Pretreatment composition comprises an acidic compound selected from organic an/or inorganic acids or their mixtures and at least one physiologically compatible hair conditioning agent selected from oily substances, non-ionic substances, cationic amphiphilic ingredients and/or cationic polymers or their mixtures.

## Description

### Technical field

This invention relates to a pre-treatment composition and, to a process for application of the said composition, which has an acidic pH and is applied to hair, especially damaged hair, before colouring hair with colouring agents containing acidic, anionic, direct dyes in order to improve the colour intensity and at the same time to achieve durable hair colour.

### Background

Hair colouring is a common practice for ages. Oxidative colouration has been widely used for achieving durable, brilliant hair colour. Direct dyes, mainly of cationic character, have also been found their applications for colouring hair for many years. The colours so achieved are brilliant but often lacking durability. Recently, anionic direct dyes have been found to be very powerful for changing hair colour permanently and to achieve long lasting, brilliant colours. The colouring agents with anionic dyes are so formulated that the optimum conditions are realised for achieving the highest dyestuff penetration into hair. European patent application with laid open number EP 1 022 014 describes such compositions comprising anionic dyestuffs, solvents, as aid to enhance penetration of said dyestuffs, and a buffer solution to adjust the pH of the dyeing agent in the range from 2 to 6. Products are found on the professional hair dressing market applying this technology.

US 5,601,620, as well, discloses hair colouring agents with acid dyes, an organic solvent and at least one polysiloxane as a conditioner. The dyeing compositions disclosed here are having a pH in the range of 1.5 - 4.5.

In practice, number of difficulties are observed when colouring human hair with colouring agents containing anionic, acidic, direct dyes. One of them is variation of colouring ability of agents with the physical status of the hair. Good colouring performance is usually obtained with healthy, natural hair, whereas the colouring performance is not always found to be satisfactory in the case of damaged, chemically processed hair and especially those of bleached hair. The reason underlying in performance difference may be that, electrostatic properties (anionic and cationic charge density and their distribution in the hair) of hair is highly altered with those chemical processes which hair is exposed to before colouring hair with acidic dyestuffs. In other words, hair is electro-statically so differed that colour uptake is not optimum and homogenous and, consequently, durability of the colour achieved is not satisfactory. In the above mentioned two patent applications those problems are not mentioned at all and, certainly, no alternative solutions are suggested.

In order to solve the aforementioned problem, it has surprisingly been found out that before carrying out colouration, application of a pre-treatment resulted in enhanced penetration of dyestuffs and therefore, intensive and even colouration of hair is obtained. The colour so achieved has shown excellent durability at the same time.

In number of previous patent applications, pre-treatment compositions are described to be used in hair colouring process either before oxidative colouration or with colouring agents containing anionic direct dyes. JP 2000229821 (abstract) describes treating agents for acidic hair dyes to be used before or after colouration. The said aqueous composition is having a pH value between 2.0 - 6.0 and containing at least one organic or inorganic acid at a concentration not less than 3.0% by weight. The acidic compounds preferred are alpha hydroxy acids such as glycolic acid. No disclosure is made about using additional hair care ingredients in the said pre-treatment composition.

JP 10182373 (abstract) describes pre-treating agent comprising 1-50% by weight of an organic solvent and an organic or an inorganic acid and having a pH of 1 - 5. The said composition is left on hair for 5 - 30 minutes at 30 - 60°C and the hair is washed before application of the dyeing agent. In this application nothing is said about the use of additional hair care agents and in addition longer processing time is preferred with the said pre-treatment agent at elevated temperatures.

WO 02/02062 describes use of salts for improving the absorption qualities of anionic direct dyes. The salts of organic or inorganic acids, which can be added to dyeing agents, can also be used in a pre-treatment formulated at an acidic pH. In this document special attention is paid to elevation of the dye uptake by addition of salts into the dyeing agent. In the document preference is given to that, salts are added to the colouring agents and using salts as pre-treatment is only optionally suggested. The document is furthermore silent about the use of for example conditioners in, if preferred, pre-treatment composition.

JP 102 919 19 (abstract) describes pre-treatment compositions to be used in colouring processes with acidic dyes and as well with oxidative dyes. The compositions described in the document contain keratin hydrolysates and its derivatives such as quaternary ammonium salts. The document does not describe the use of conditioners in pre-treatment at all.

An alkaline pre-treatment composition is described in JP 2000 169 344 (abstract). The composition furthermore contains organic solvent and has a pH value in the range of 7.5 to 11.0. It has also been suggested to add reducing agents into the compositions to enhance colouring ability and durability.

JP 2002 029 947 (abstract) describes pre-treatment composition for acidic dyes for improving dyeing ability and fastness. The compositions described therein contain at least one kind of cationic polymer selected from the group consisting of an amine-base and a quaternary ammonium group. No information can be found in the document about the pH of the compositions. Moreover, the document is silent about the use of conditioning agents other than those of cationic polymers.

### Detailed description of the invention

The pre-treatment composition, to be used before colouring hair with colouring agents containing acidic dyes, found surprisingly being an excellent aid for improving colouring intensity and achieving excellent durability. The pre-treatment composition is characterised in that
- it has pH value from 1.5 to 5, preferably 2 to 4 and most preferably 2 - 3.5, and comprising
- an organic and/or inorganic acid and/or their mixtures and
- at least one physiologically compatible hair conditioning agent selected from oily substances, non-ionic substances, cationic amphiphilic compounds, cationic polymers or their mixtures.

Optionally addition of salts of organic and/or inorganic into the said composition increases the colour uptake as well. At the same time, with the use of pre-treatment, hair is made easily combable. In the colouring process with direct dyes, colouring agent must be applied to whole hair including lengths. Since combability of hair is improved with the use of pre-treatment, this contributes to easy and uniform application of colouring agent and consequently results in obtaining even colour results.

The process of colouring hair with acidic direct dyes with the use of pre-treatment composition described here involves two step. Those are:
1- Application of the pre-treatment composition and subsequently
2- colouring hair with colouring agent containing acidic dyestuffs.

In this process, between the two steps, although it is not essential, hair may be towel dried or air dried or dried with an electrical drier. Hair may also be optionally rinsed off and with or without (towel) drying and/or drying with an electrical drier colouring process can be continued. When colouring agent is applied directly after application of the pre-treatment composition, an up to 5 minute processing time should be allowed before applying colouring agents.

It has further been found out that drying hair before colouration, with and/or without processing time after application of the pre-treatment, improves evenness of colour further thus obtained, as no dilution of colouring agents takes place during the process.

It has been found out that amount of pre-treatment applied to hair is an important parameter. Special attention must be paid to even application of pre-treatment onto hair so that hair is saturated with the pre-treatment composition. Typical amounts of 20 - 35 g are found to be satisfactory for medium length hair. This can also be expressed in a pre-treatment amount to hair ratio which should be typically 0.5:1 to 1:1 and in any event in the range from 0.3:1 to 2:1 by weight.

For adjusting the pH of the said pre-treatment composition, following ingredients can be used: Organic acids such as citric acid, lactic acid, tartaric acid, malic acid, maleic acid, fumaric acid, levulinic acid, butyric acid and hydroxy butyric acids, valeric acid, oxalic acid, succinic acid, mandelic acid, glycolic acid, glucuronic acid, propionic acid, salicylic acid or acetic acid or inorganic acids such as hydrochloric acid, phosphoric acid, sulphuric acid, nitric acid. Concentration of the organic and/or inorganic acids or their mixtures should be adjusted in a way that pre-treatment composition so obtained has a pH value between 1.5 and 5, preferably 2 - 4 and more preferably 2 - 3.5. Typically concentration for acids can be 0.2 - 30% by weight, preferably 0.5 - 15% by weight, more preferably 0.5 - 8% by weight and most preferably 0.5 - 6% by weight. The pH of the pre-treatment composition can also be adjusted to the required pH by using alkaline solution such as sodium hydroxide, potassium hydroxide or their salts with those acids mentioned above in the case that at the selected acid concentration pH of the composition is lower than that of the aimed value. In the case that salts are used for adjusting pH, concentration of salts should not exceed 3% by weight.

The pre-treatment composition of this invention comprises hair conditioning agents. Conditioning agents can be selected from oily substances, non-ionic substances, cationic amphiphilic ingredients, cationic polymers or their mixtures. Oily substances are selected from such as silicone oils either volatile or non-volatile, natural and synthetic oils. Among silicone oils those can be added to the pre-treatment composition include dimethicone, dimethiconol, polydimethylsiloxane, DC fluid ranges from Dow Corning, natural oils such as olive oil, almond oil, avocado oil, weizenkeim oil, ricinus oil and the synthetic oils, such as mineral oil.

Non-ionic conditioning agents may be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula I or II, respectively,

R₁ CO (O CH₂ CH₂)ₙ OH formula I

R₁ CO (O CH₂ CH₂)ₙ O OC R₂ formula II

where R₁ and R₂ are independent from each other saturated, unsaturated or branched or non-branched alkyl chain with 7 to 21 C atoms and n is typically 2 - 100.

Pre-treatment composition can contain cationic amphiphilic conditioning ingredients according to the formula III, but not limited to. 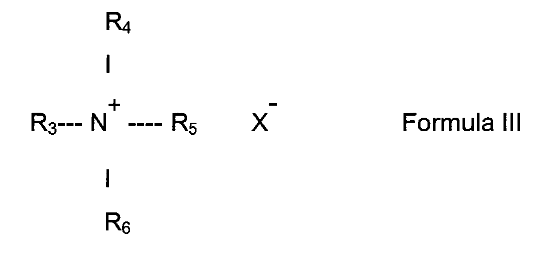 where R₃ is a saturated or unsaturated, branched or non-branched alkyl chain with 8-22 C atoms or

R₇ CO NH (CH₂)ₙ

where R₇ is saturated or unsaturated, branched or non-branched alkyl chain with 7-21 C atoms and n has typical value of 1 - 4 or

R₈ CO O (CH₂)ₙ

where R₈ is saturated or unsaturated, branched or non-branched alkyl chain with 7-21 C atoms and n has typical value of 1 - 4, and
R₄ is H or unsaturated or saturated, branched or non-branched alkyl chain with 1 - 22 C atoms or

R₇ CO NH (CH₂)ₙ

or

R₈ CO O (CH₂)ₙ

where R₇, R₈ and n are same as above.

R₅ and R₆ are H or lower alkyl chain with 1 to 4 Carbon atoms, and X is typically chloride, bromide, methosulfate.

Typical examples of those ingredients are cetyl trimethly ammonium chloride, stear trimonium chloride, dipalmitoyl dimonium chloride, distearyl dimethyl ammonium chloride, stearamidopropyl trimonuim chloride, dioleoylethyl dimethyl ammonium methosulfate, dioleoylethyl hydroxyethylmonium methosulfate.

Pre-treatment composition can also contain cationic polymers as conditioning agents. Those are cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride. Furthermore, chitosan and chitin can also be included in the compositions as cationic natural polymers.

Typical concentration range for any of the conditioners mentioned above can be 0.01 - 5% by weight, preferably 0.03 - 2% by weight and more preferably 0.05 - 0.75% by weight.

Pre-treatment composition can be in the form of a solution, with either transparent, semitransparent or non-transparent milky appearance, gel type of preparation again with either transparent, semitransparent or non-transparent appearance, or an emulsion. The compositions can have viscosity values between 1 mPa.s to 40,000 mPa.s, preferably 1 mPa.s to 20,000 mPa.s and more preferably 1 mPa.s to 15,000 mPa.s and most preferably 1 mPa.s to 10,000 mPa.s measured at 20°C with Brookfield viscosimeter with, for example, Spindle 4 at 10 rpm. The viscosity values are read after 30 seconds from the start of the measurement. In the selection of the viscosity, special attention must be paid to the way of application and packaging to be used. In other words, for example, when a spray application is preferred, the viscosity of preparations must be low enough allowing spraying.

Pre-treatment composition can be packed into a bottle with a nozzle which enables easy application or with a spray device (pump spray) or with a pump which enables dispensing the composition in the form of liquid or foam (pump foamer). Composition may also be offered in an aerosol bottle from which the composition is dispensed as a foam. In the aerosol form, dispensing as a spray may also find its applications in the daily practice. In the case that aerosol form is preferred, suitable propellant gas or mixtures must be added to the composition to make dispensing in the preferred form possible.

Transparency of the preparations can be adjusted by altering the type and/or concentration of the ingredients, especially conditioners, as mentioned above and/or by addition of an opacifier such as Styrene/PVP Copolymer known with trade name Antara 430 from ISP at a concentration up to 1.0% by weight.

For gel type preparations, compositions may contain non-ionic polymers such as hydroxyethylcellulose, hydroxypropylclellulose, xanthan gum, xyloglucan, polyvinylalcohol, polyvinylpyrrolidone or their derivatives.

Anionic polymers can also be used as thickeners. Typical example of those is acrylate type of polymers know with the trade name Carbopol from Goodrich.

In the case that pre-treatment composition is an emulsion, the composition may then contain fatty alcohols with 12 to 22 carbon atoms and non-ionic emulsifier such as ethoxylated fatty alcohols with general formula (IV)

R₉ (O CH₂ CH₂)ₙ OH formula IV

where R9 is saturated or unsaturated, branched or non-branched alkyl chain with 8-22 C atoms and n has typical value of 2 -100.

The pre-treatment compositions may contain organic solvents such as ethanol, propanol, isopropanol, benzyl alcohol, benzyloxyethanol, alkylene carbonates such as ethylene carbonate and propylene carbonate, phenoxyethanol, butanol, isobutanol, cyclohexane, cyclohexanol, hexyleneglycol, ethylenecarbonate, ethyleneglycol monoethylether, ethylene glycol monobutyl ether, ethylene glycol monophenyl ether, 1-phenylethylalcohol, 2-phenylethylalcohol, o-methoxyphenol.

Concentration of organic solvents in the pre-treatment composition should not exceed 30% by weight, preferably 5% by weight.

Solubilizers may be added to the compositions especially when oily substances are chosen as conditioning agents. Typical solubilizers may be hydrogenated castor oil known with the trade mark Cremophor RH series from BASF, non-ionic surfactants, such as ethoxylated fatty alcohols, alkyl glucoside, or amphoteric surfactants such as betaine type i.e. cocamidopropyl betaine. Typical concentration of the solubilizers can be in the range of 0.01 - 2% by weight, preferably 0.1 - 1% by weight.

Pre-treatment composition may contain additional cosmetic ingredients such as fragrance, preservative, sequestering agents, UV filters.

Following are the examples to illustrate the invention but are not to be interpreted as limiting it.

### Example 1

| Pre-treatment solution with transparent appearance | |
|---|---|
| | % by weight |
| Cetrimonium chloride | 0.25 |
| Cremophor RH 40¹ | 0.25 |
| Lactic acid | 3.5 |
| Sodium hydroxide | q.s. to adjust the pH to 2.8 |
| Fragrance | q.s. |
| Water ad | 100 |

| | |
|---|---|
| ¹: PEG-40 hydrogenated castor oil | |

All ingredients are added to water one by one and dissolved, except fragrance which is mixed first with Cremophor RH 40 and aqueous solution of cetrimonium chloride and then added to the mixture. The composition so obtained is a clear solution with a viscosity less than 100 mPa.s. at 20°C. When desired the appearance can be altered by addition of Antara 430 at various concentrations.

The composition can be provided in a bottle with an application nozzle, in a bottle with a spraying device or in an aerosol form from which dispensing in a foam or spay is possible. In the case that aerosol type product is preferred, appropriate propellant gas must be used.

### Colouring test:

Healthy and oxidatively bleached caucasian hair (original colour is medium blond) tresses are prepared as hair samples for colouring tests. The tresses are bleached 3x with commercially available agents (Oxycure Platin from Goldwell GmbH). Elumen RR @ all (commercial product of Goldwell GmbH) is used as colouring agent in all tests unless otherwise stated. In each test run two tresses used and one as being control, coloured without pre-treatment (see below), and to the other first a pre-treatment composition as described above is applied. The applied amount of the pre-treatment solution is equal to the weight of the hair tress (1:1 ratio of pre-treatment : hair by weight). After drying tresses with and electical dryer, colouring agent is applied and processed for 20 minutes at 40°C, then rinsed off and dried with an electrical drier. Colour measurements are carried out with a commercial equipment (Minolta CR-200) in order to determine the difference in colour nature and intensity. Delta E values are calculated from the L, a and b values measured before and after colouration. Results are presented in Table I.

**Table I**

| | Pre-treatment | Delta E |
|---|---|---|
| Healthy hair | - | 57.0 |
| | + | 58.5 |
| Bleached hair | - | 61.0 |
| | + | 72,5 |

As can be seen from the delta E values, application of the pre-treatment on healthy, undamaged, hair has only a slight effect, whereas the effect is very much elevated on bleached, chemically damaged hair.

For testing the effect of amount of the pre-treatment composition applied to the hair, same as above oxidatively damaged hair tresses are taken and coloured in the same way as described above. In the process only the amount of applied pre-treatment is varied as 0.5, 1.0 (same as above) and 2.0 times of the weight of the hair tress. Colour measurements are carried out and delta E values are calculated in the same way as above. Results are presented in Table II.

**Table II**

| Pre-treatment : Hair ratio | Delta E |
|---|---|
| 0:1 | 61.0 |
| 0.5:1 | 72.6 |
| 1:1 | 72.5 |
| 2:1 | 73.5 |

The results show clearly that even at lower application amounts of pre-treatment (pre-treatment : hair ratio of 0.5:1) excellent colour intensity is achieved.

For testing durability, tresses coloured in the same way as described above with and without pre-treatment are subjected to a wash test. The wash test is carried out as placing the tresses in a 10% by weight solution of shampoo suitable for coloured hair (Definition Color and Highlight Shampoo, commercial product of Goldwell GmbH) and shaken in a water bath at 30°C and at approximately 50 rpm for 15 minutes. The tresses are then taken out and rinsed with running water at around same temperature and after drying colour measurements are carried out. The delta E values are calculated by taking the L, a and b values before colouring the tress as a reference. The same procedure is repeated for the second time in the same way. This test is only carried out on chemically damaged hair tresses and with 2 different colours from the same brand commercially available from Goldwell GmbH.

**Table III**

| | NB@5 | RR@all | | |
|---|---|---|---|---|
| | | Delta E | | |
| | without | with | without | with |
| After colouring | 45 | 52 | 61 | 72,5 |
| 15 min shaking | 40 | 48 | 55 | 68,5 |
| 30 min shaking | 38 | 45 | 52 | 65 |

As results show clearly, not only the colour intensity but also the durability is very much enhanced by application of pre-treatment before colouring hair with acidic dyestuff containing colouring agents.

Similar results are as well obtained with the following examples.

### Example 2

| Pre-treatment solution with semitransparent appearance | |
|---|---|
| | % by weight |
| Dioleoylethyl hydroxyethylmonium methosulfate | 0.25 |
| Cremophor RH 40 | 0.25 |
| Citric acid | 3.60 |
| Sodium hydroxide | q.s. to adjust the pH to 2.2 |
| Fragrance | q.s. |
| Water ad | 100 |

The solution is prepared as described in example 1. The composition thus obtained is a slightly turbid solution.

Similarly, the composition can be provided in a bottle with an application nozzle, in a bottle with a spraying device or in an aerosol form from which dispensing in a foam or spay is possible. In the case that aerosol type product is preferred, appropriate propellant gas or mixtures must be added. Intensive, brilliant and durable colours are achieved by application of this composition before colouring hair with acidic dyes containing colouring agents.

### Example 3

| Gel type pre-treatment | |
|---|---|
| | % by weight |
| Polyquaternium-6 | 0.25 |
| Hydroxyethylcellulose | 1.0 |
| Cremophor RH 40 | 0.1 |
| Citric acid | 3.10 |
| Sodium hydroxide | q.s. to adjust the pH to 2.5 |
| Fragrance | q.s. |
| Water ad | 100 |

The composition is prepared by dissolving citric acid in water and subsequently, the mixture of Cremophore RH 40, fragrance and Polyquaternium -6 is added. Then hydroxyethylcellulose is dissolved in the mixture and pH is adjusted to 2.5 with sodium hydroxide solution. The gel type preparation thus obtained is clear and have viscosity of 8,720 mPa.s measured at 20°C with Brookfield viscosimeter Spindle 4 at 10 rpm.

The composition can be provided in a bottle and/or tube with application nozzle. Use of the composition before colouring hair with colouring agents containing acidic dyes resulted in improvement in colour intensity, gloss and durability.

### Example 4

| Emulsion Pre-treatment | |
|---|---|
| | % by weight |
| Cetearyl alcohol | 2.0 |
| Ceteareth 20 | 0.25 |
| Sodium hydroxide | q.s. to adjust the pH to 3.0 |
| Lactic acid | 3.5 |
| Fragrance | q.s |
| Water ad | 100 |

Emulsion is prepared by melting cetearyl alcohol and ceteareth 20 in lactic acid solution in water at about 70°C and homogenizing for a short time at 2000 rpm. After cooling down, fragrance is added and pH is adjusted to 3.0 by addition of sodium hydroxide solutionThe emulsion thus obtained free flowing white mass, with a viscosity of less than 100 mPa.s measured at 20°C with Brookfield viscosimeter Spindle 1 at 10 rpm. The composition can be provided in a bottle and/or tube with application nozzle. Easy application of the colouring agents after applying this composition, especially to the lengths by combing through is achieved as well as intensive, brilliant and durable colour.

## Claims

1. Pre-treatment composition to be used before colouring hair with colouring agents containing acidic dyestuff **characterised in that** it has a pH in the range from 1.5 to 5 and comprises organic and/or inorganic acids or their mixtures and at least one physiologically compatible hair conditioning agent.

2. Pre-treatment composition according to claim 1, has a pH in the range from 2 to 4.

3. Pre-treatment composition according to claim 1, has a pH in the range from 2 to 3.5.

4. Pre-treatment composition according to claims 1 to 3, comprises organic and/or inorganic acids or their mixtures at a concentration of 0.2 - 30% by weight.

5. Pre-treatment composition according to claims 1 to 3, comprises organic and/or inorganic acids or their mixtures at a concentration of 0.2 - 8% by weight.

6. Pre-treatment composition according to claims 1 to 3, comprises organic and/or inorganic acids or their mixtures at a concentration of 0.2 - 6% by weight.

7. Pre-treatment composition according to any of the preceding claims comprises lactic acid as the acidic compound.

8. Pre-treatment composition according to claims 1 to 7, comprises at least one physiologically compatible hair conditioning agent selected from oily substances, non-ionic substances, cationic amphiphilic compounds, cationic polymers or their mixtures.

9. Pre-treatment composition according to claim 8, hair conditioning agent is an oily substance.

10. Pre-treatment composition according to claim 8, hair conditioning agent is a non ionic substance.

11. Pre-treatment composition according to claim 8, hair conditioning agent is a cationic amphiphilic compound with a general formula 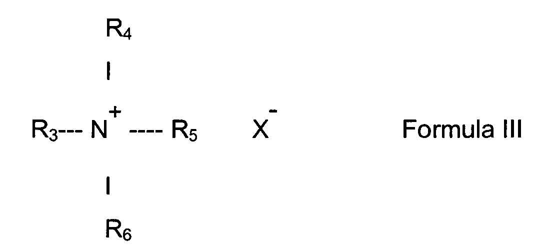 where R₃ is a saturated or unsaturated, branched or non-branched alkyl chain with 8-22 C atoms, or R₇ CO NH (CH₂)ₙ where R₇ is saturated or unsaturated, branched or non-branched alkyl chain with 7-21 C atoms and n has typical value of 1 - 4, or
R₈ CO O (CH₂)ₙ where R₈ is saturated or unsaturated, branched or non-branched alkyl chain with 7-21 C atoms and n has typical value of 1 - 4
and
R₄ is H or unsaturated or saturated, branched or non-branched alkyl chain with 1 - 22 C atoms or R₇ CO NH (CH₂)ₙ or R₈ CO O (CH₂)ₙ
and
R₅ and R₆ are independent from each other H or lower alkyl chain with 1 to 4 Carbon atoms, and X is typically chloride, bromide, methosulfate.

12. Pre-treatment composition according to claim 8, hair conditioning agent is a cationic polymer.

13. Pre-treatment composition according to claims 8 to 12, comprises conditioning agents at a concentration of 0.01 - 5% by weight.

14. Pre-treatment composition according to claims 8 to 12, comprises conditioning agents at a concentration of 0.01 - 2% by weight.

15. Pre-treatment composition according to claims 8 to 12, comprises conditioning agents at a concentration of 0.03 - 0.75% by weight.

16. Pre-treatment composition according to any of the preceding claims, comprises organic solvents at a concentration of 30% by weight or less.

17. Pre-treatment composition according to any of the preceding claims comprises salts of organic and/or inorganic acids or their mixtures at a concentration of 3% by weight or less.

18. Pre-treatment composition according to any of the preceding claims, has viscosity from1 mPa.s to 40,000 mPa.s, preferably from 1 mPa.s to 20,000 mPa.s and more preferably from 1 mPa.s to 15,000 mPa.s and most preferably from1 mPa.s to 10,000 mPa.s measured at 20°C with Brookfield viscosimeter with for example Spindle 4 at 10 rpm.

19. Pre-treatment composition according to any of the preceding claims, contains additional cosmetic ingredients such as fragrance, preservative, sequestering agents, UV filters.

20. Pre-treatment composition according to any of the preceding claims, is in the form of a solution with either transparent, semitransparent or milky appearance, in the form of a gel either transparent, semitransparent or milky appearance and in the form of an emulsion and packed into a bottle with an application nozzle, with a spraying device or into a dispenser which allows dispensing as a liquid or a foam and in an aerosol form which allows dispensing as a spray or a foam.

21. Process for colouring hair with colouring agents containing acidic dyestuff **characterised in that** pre-treatment composition according to any of the preceding claims is applied first to the hair and optionally left on the hair up to 5 minutes than colouring agent is applied.

22. Process according to claim 21 **characterised in that** hair is towel dried or air dried or dried with an electrical drier after application of pre-treatment composition and subsequently colouring agent is applied.

23. Process according to claims 21 and 22 **characterised in that** applied amount of pre-treatment to hair ratio is in the range from 0.3:1 to 2:1 by weight, preferably 0.5:1 to 1:1 by weight.

24. Pre-treatment composition and process according to any of the preceding claims **characterised in that**, pre-treatment composition is applied especially to oxidatively damaged and/or bleached hair before colouring hair with colouring agents containing acidic dyestuff.
